# EUROPÄISCHE PATENTSCHRIFT

(11) **EP 1 651 157 B1**
(45) Veröffentlichungstag und Bekanntmachung des Hinweises auf die Patenterteilung: **02.01.2013**
(21) Anmeldenummer: 04732093.2
(22) Anmeldetag: 11.05.2004
(51) Int. Cl.: A61F 13/64, A61F 13/56

(54) **WEGWERFWINDEL MIT HÜFTGÜRTEL**
DISPOSABLE NAPPY WITH A HIP BAND
COUCHE JETABLE AVEC CEINTURE DE HANCHES

(30) Priorität: 06.08.2003 DE 10337537
(43) Veröffentlichungstag der Anmeldung: 03.05.2006
(73) Patentinhaber: Paul Hartmann Aktiengesellschaft, 89522 Heidenheim (DE)
(72) Erfinder: RÖHRL, Wolfgang, 89542 Herbrechtingen (DE); STUPPERICH, Hans-Peter, 89552 Heidenheim (DE); BANDORF, Matthias, 97422 Schweinfurt (DE)
(74) Vertreter: Friz, Oliver
(86) Internationale Anmeldenummer: PCT/EP2004/005012
(87) Internationale Veröffentlichungsnummer: WO 2005/023161

(56) Entgegenhaltungen:
- EP-A- 0 206 208
- EP-A- 1 110 529
- EP-A- 1 269 949
- EP-B- 0 941 041
- DE-A- 19 732 499
- US-A- 5 318 555
- US-A1- 2001 034 512
- US-A1- 2002 045 881

## Beschreibung

Die Erfindung betrifft eine Wegwerfwindel, insbesondere zur Inkontinentenversorgung, mit einem eine in Umfangsrichtung geschlossene Hüftöffnung der Windel bildenden Hüftgürtel und mit den weiteren Merkmalen des Oberbegriffs des Anspruchs 1.

Eine derartige Wegwerfwindel ist bekannt aus EP 1 110 529 A1. US 2001/0034512 A1 oder US 2002/45881 A1 oder EP 1 269 949 A2 zeigen weitere Wegwerfwindeln mit einem Hüftgürtel.

Derartige Wegwerfwindeln sind vielfach bekannt geworden. Sie bieten den Vorteil, dass ein Benutzer beim Anlegen der Windel zunächst den Hüftgürtel um die Hüften herumlegt, und üblicherweise im Bauchbereich schließen kann. Währenddessen hängt der üblicherweise mit seinem Rückenbereich am Gürtel befestigte Windelhauptteil der Wegwerfwindel lose nach unten. Der Benutzer ergreift dann nach dem Schließen des Hüftgürtels das freihängende Ende des Windelhauptteils und führt den Windelhauptteil von hinten zwischen den Beinen hervor, um den Windelhauptteil mit seinem freien Längsende am Hüftgürtel innen oder außen lösbar festzulegen. Hierfür sind die ersten Verschlussmittel vorgesehen. Es versteht sich, dass das Anlegen der Wegwerfwindel auch so ausgeführt werden kann, dass nach dem Anlegen und Schließen des Hüftgürtels der frei nach unten hängende Windelhauptteil von vorn nach hinten zwischen den Beinen eines Benutzers hindurchgeführt und solchenfalls mit seinem Rückenbereich am Hüftgürtel lösbar befestigt werden kann. Es sind aber auch Wegwerfwindeln bekannt geworden, bei denen der Windelhauptteil ganz vom Hüftgürtel lösbar ist, so dass insbesondere bei stark pflegebedürftigen und immobilen Benutzern eine weitgehende Flexibilität bei der Handhabung der Wegwerfwindel gewährleistet ist.

Bei einigen der vorausgehend erwähnten Druckschriften werden beidseits des Windelhauptteils zwei separate in Querrichtung ausladende Materialabschnitte zur Bildung des Hüftgürtels angefügt. Da während des bestimmungsgemäßen Gebrauchs der Windel hohe Kräfte im Gürtel auftreten, besteht das Problem der Einleitung und Aufnahme dieser Kräfte in den Windelhauptteil.

Der vorliegenden Erfindung liegt die Aufgabe zugrunde, eine Wegwerfwindel der gattungsgemäßen Art für einen einfachen Gebrauch weiterzubilden. Des Weiteren soll ein hierfür geeignetes Herstellverfahren geschaffen werden.

Diese Aufgabe wird durch eine Wegwerfwindel mit den Merkmalen des Anspruchs 1 gelöst.

Es wird also ein in Querrichtung oder Hüftumfangsrichtung einstückig durchgehender Materialabschnitt an den Windelhauptteil zur Bildung des Hüftgürtels angefügt. Aufgrund der flächenhaften Anfügung werden die Zugkräfte nicht in den Windelhauptteil eingeleitet sondern können ganz von dem reißfesten Gürtelmaterial aufgenommen werden. Dies eröffnet bei der Auswahl der den Hauptteil bildenden Chassismaterialien eine größere Flexibilität. Außerdem sind die Anforderungen an die Stabilität der Anfügung des Gürtels an den Hauptteil geringer. Der den Gürtel bildende Materialabschnitt kann, wie später anhand des erfindungsgemäßen Herstellungsverfahrens erörtert werden wird, als ein einziger Längsabschnitt einer in der Maschinenrichtung zugeführten Flachmaterialbahn erhalten werden.

In weiterer Ausbildung des Erfindungsgedankens erweist es sich als vorteilhaft, wenn der Hüftgürtel in der beidseits gefalteten Konfiguration lösbar fixiert ist, so dass er sich innerhalb der schnelllaufenden Herstellungsmaschine nicht unbeabsichtigt entfaltet oder aufflattert. Die Faltung und lösbare Fixierung des Hüftgürtels in der gefalteten Konfiguration ermöglicht in vorteilhafte Weise die Anfügung des sehr langen Hüftgürtels an den Hauptteil in der Herstellungsmaschine.

In Weiterbildung dieses Erfindungsgedankens erweist es sich als vorteilhaft, wenn die lösbare Fixierung durch Fügestellen oder Fügebereiche zwischen den aufeinandergefalteten Teilabschnitten des den Hüftgürtel bildenden Materialabschnitts gebildet ist. Es wäre aber auch möglich, das die gefaltete Konfiguration durch sonstige Haltemittel, etwa einen lösbaren Tapeabschnitt, lösbar fixiert ist.

Die vorerwähnte und vorzugsweise in einem Zug zu lösende Fixierung der aufeinander gefalteten Teilabschnitte des an den Hauptteil angefügten den Hüftgürtel bildenden Materialabschnitts kann beispielsweise durch kalte Verprägung, oder durch Verprägung unter Anwendung von Temperatur (Thermoverschweißung), durch Vernadelung, insbesondere Heißvernadelung, oder durch Ultraschallverschweißung oder Laserverschweißung oder ähnliche gleichwirkende Fügemethoden erreicht werden.

Im einfachsten Fall ist der Materialabschnitt beidseits der Längsmittelachse um eine Falzlinie auf sich selbst gefaltet, so dass zwei Teilabschnitte aufeinander liegen bzw. gegeneinander anliegen. Vorzugsweise ist der Materialabschnitt aber um wenigstens zwei Falzlinien auf sich selbst gefaltet, so dass eine im Schnitt Z-förmige Konfiguration entsteht. Nach einer weiteren bevorzugten Ausführungsform sind die Materialabschnitte um drei Falzlinien auf sich selbst gefaltet. Nach einer weiteren bevorzugten Ausführungsform sind die Materialabschnitte um vier Falzlinien auf sich selbst gefaltet.

Nach einer weiteren bevorzugten Ausführungsform der erfindungsgemäßen Gürtelwindel steht der einstückige Materialabschnitt in gefalteter Konfiguration mit einem Anfassbereich an seinem freien Ende über einen Längsseitenrand des Windelhauptteils in Querrichtung vor. Der Anfassbereich kann insbesondere vom jeweiligen freien Ende des Hüftgürtels gebildet sein.

Vor der Entfaltung des einstückigen Materialabschnitts sind die Anfassbereiche vorzugsweise in Querrichtung nach außen gewandt, also voneinander und von einer Längsmittelachse des auf einer ebenen Unterlage ausgebreiteten Windelhauptteils voneinander weggewandt, so dass sie auf bequeme Weise mit der linken Hand eines Benutzers von links und mit der rechten Hand von rechts ergreifbar sind. Die Gürtelwindel erweist sich insbesondere bei der Anwendung bei stark pflegebedürftigen Personen als besonders vorteilhaft. So wird die Gürtelwindel beispielsweise häufig an pflegebedürftige Patienten angelegt, während diese sich in Seitenlage befinden. Hierbei muss der über den Hauptteil seitlich überstehende Materialabschnitt unter dem Patienten hindurch geführt werden. Dieser Vorgang des unter dem Patienten Hindurchführens ist mit dem lösbar fixierten gefalteten Materialabschnitt, welcher den Gürtel bildet, nun wesentlich vereinfacht.

Die lösbare Fixierung der aufeinander gefalteten Teilabschnitte des Materialabschnitts aneinander und möglicherweise auch an dem Hauptteil ist vorzugsweise durch mehrere im Wesentlichen punktförmige Fügestellen ausgebildet. Eine punktförmige Fügestelle der vorstehend erwähnten Art bedeutet, dass die Fügestelle eine Fläche (in Projektion auf die X-Y-Ebene des Hauptteils) von weniger als 5 mm², insbesondere von weniger als 2 mm² und weiter insbesondere von weniger als 1 mm² aufweist. Die Fügestellen müssen nicht streng punkt- oder kreisförmig sein. Denkbar und vorteilhaft sind auch von der Punkt- oder Kreisform abweichende Formen wie Dreieck-, Viereck-, Vieleck-, oder Ovalformen. Vorzugsweise ist die lösbare Fixierung der aufeinander gefalteten Teilabschnitte der Materialabschnitte aneinander durch thermisch oder durch Ultraschall erzeugte, vorzugsweise punktförmige Fügestellen ausgebildet.

Die Erstreckung des den Hüftgürtel bildenden Materialabschnitts im entfalteten Zustand in Querrichtung über den Längsrand des Hauptteils hinaus beträgt wenigstens 200 mm, insbesondere wenigstens 300 mm, insbesondere wenigstens 400 mm, insbesondere wenigstens 500 mm und weiter insbesondere wenigstens 600 mm. Seine Erstreckung in Längsrichtung beträgt 30 bis 120 mm, insbesondere 30 bis 100 mm, insbesondere 30 bis 80 mm, insbesondere 30 bis 75 mm, insbesondere von 44 bis 70 mm, insbesondere von 40 bis 65 mm und weiter insbesondere von 40 bis 60 mm aufweist

Der einstückige Materialabschnitt ist in vorteilhafter Weise unlösbar an eine Außenseite des Hauptteils angefügt. Diese Anfügung kann in an sich beliebiger Weise ausgeführt sein. Der einstückige Materialabschnitt kann indessen in vorteilhafter Weise mittels eines nur bereichsweisen Kleberauftrags an den Windelhauptteil angefügt sein. Insbesondere erweist es sich als vorteilhaft, wenn der Kleber nicht bis zu dem Rand der gegeneinander anliegenden flächenhaften Erstreckung des Materialabschnitts und des Hauptteils aufgebracht ist, so dass ein Randumfangsbereich dieses Verbunds kleberfrei bleibt. Dies hat den Vorteil, dass solchenfalls beim Laminieren kein Kleber zwischen den Lagen nach außen gedrückt wird.

Die vorliegende Erfindung hat auch ein Verfahren zum Herstellen einer Wegwerfwindel der erfindungsgemäßen Art zum Gegenstand. Dieses Verfahren ist gekennzeichnet durch die folgenden Verfahrensschritte:
- Zuführen einer endlosen Flachmaterialbahn zur Bildung von Materialabschnitten für die Herstellung des Hüftgürtels,
- Falten der Flachmaterialbahn um in Längsrichtung verlaufende Falzlinien, wobei die Flachmaterialbahn beidseits einer Längsmittelachse auf sich selbst gefaltet wird,
- Abtrennen von Längsabschnitten der gefalteten Flachmaterialbahn zur jeweiligen Bildung eines einstückigen Materialabschnitts für die Herstellung des Hüftgürtels und
- unlösbares Fixieren des Materialabschnitts an einem Windelhauptteil.

Bevorzugte Weiterbildungen des erfindungsgemäßen Verfahrens ergeben sich aus den weiteren Unteransprüchen. Insbesondere erweist es sich als vorteilhaft, wenn der einstückige den Hüftgürtel bildende Materialabschnitt mittels Kleber an den Windelhauptteil angefügt wird. Es erweist sich des Weiteren als vorteilhaft, wenn hierfür der Kleber auf die Flachmaterialbahn, von der die Materialabschnitte abgetrennt werden, in voneinander in Längsrichtung beabstandeten Bereichen aufgebracht wird. Ein jeweiliger Schnittbereich wird dabei vorzugsweise kleberfrei gehalten.

Des Weiteren erweist es sich als vorteilhaft, dass der Kleber derart bereichsweise auf die Flachmaterialbahn aufgebracht wird, dass er in Längsrichtung und vorzugsweise auch in Querrichtung von einem Rand der später abgetrennten Flachmaterialabschnitte beabstandet ist. Solchenfalls erweist es sich als vorteilhaft, wenn der Kleber getaktet auf die Flachmaterialbahn aufgebracht wird.

Der einstückige an den Hauptteil angefügte Materialabschnitt ist vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Auch Vliesstofflaminate, also insbesondere zweilagige, dreilagige oder mehrlagige Kombinationen der vorgenannten Vliesstoffe, können verwendet werden. Die Verbindung der einzelnen Lagen kann durch an sich übliche und bekannte Verfahren, beispielsweise durch thermische Fügeverfahren (Verschweißung, insbesondere Laserverschweißung, hotmelt, air-through) oder durch Ultraschallschweißverfahren erfolgen; auch die kalte Verpressung, Vernadelung, Vernähung oder Verklebung von Vliesstoffmaterialien ist denkbar. Auch die Verbindung mit textilen Geweben, Gewirken oder Gestricken, also mit eine textile Bindung im weitesten Sinne aufweisenden Materialien ist denkbar. Auch Filme aus thermoplastischen und insbesondere elastischen Materialien sind verwendbar, und zwar auch als mehrlagige Filmlaminate. Vorteilhafterweise können auch Vlies/Folienlaminate zum Einsatz kommen, die wenigstens eine Filmlage und wenigstens eine Vlieslage oder wenigstens eine Lage eines textilen Materials umfassen. Filmlagen werden insbesondere zur Realisierung von elastischen Bereichen des Hüftgürtels eingesetzt. Die Verbindung der Lagen kann wiederum durch die vorgenannten Verfahren erfolgen.

Vorzugsweise wird der Materialabschnitt zumindest abschnittsweise atmungsaktiv ausgebildet, wobei insbesondere eine Mikroporosität als vorteilhaft angesehen wird, die sowohl einen Luftaustausch als auch eine Durchlässigkeit für Feuchtigkeit in Form von Wasserdampf gestattet. Die Materialabschnitte haben in vorteilhafter Weise ein Flächengewicht von 30 bis 150 g/m².

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Windel. In der Zeichnung zeigt:
- Figur 1: eine schematische Darstellung einer erfindungsgemäßen Gürtelwindel mit gefaltetem Hüftgürtel;
- Figur 2: eine schematische Schnittansicht mit Schnittebene A-A der Gürtelwindel nach Figur 1 mit einer ersten Faltung des Hüftgürtels;
- Figur 3: eine schematische Schnittansicht mit Schnittebene A-A der Gürtelwindel nach Figur 1 mit einer zweiten Faltung des Hüftgürtels und
- Figur 4a - e: in schematischer Darstellung Zuführung, Faltung, Fixierung, Kleberbeschichtung und Schneiden einer Hüftgürtelabschnitte bildenden Flachmaterialbahn.

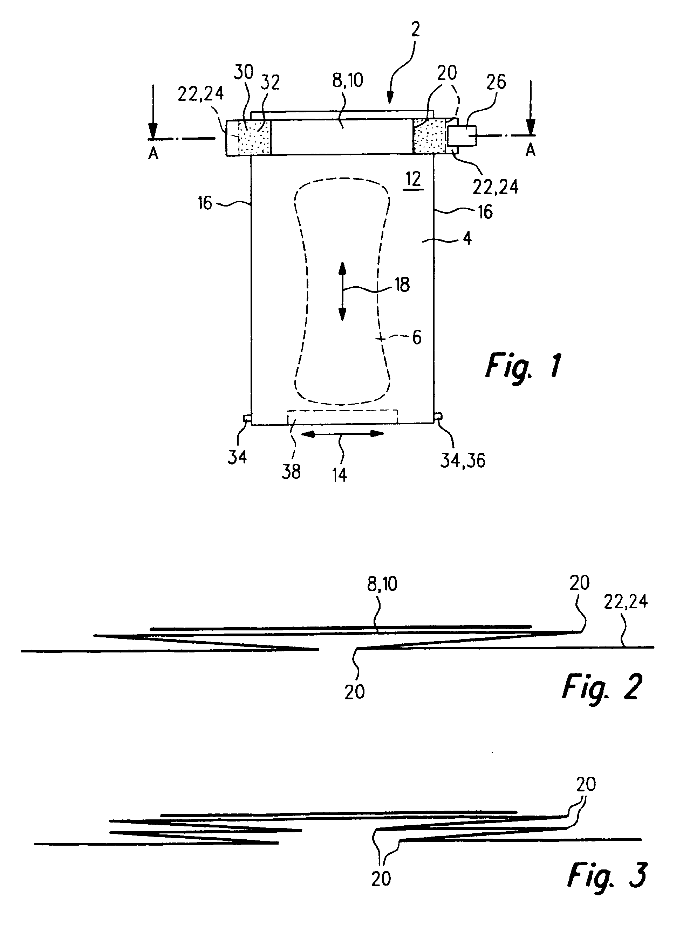

Figur 1 zeigt schematisch eine wegwerfbare Gürtelwindel 2 mit einem Hauptteil 4 und einem angedeuteten Absorptionskörper 6. An den Hauptteil 4 angefügt ist ein einstückiger Materialabschnitt 8, welcher einen Hüftgürtel 10 der Gürtelwindel bildet. Der einstückige Materialabschnitt 8 ist an eine Außenseite 12 des Hauptteils 4 auf noch näher zu beschreibende Weise unlösbar angefügt. Er erstreckt sich in Querrichtung 14 der Gürtelwindel 2 über seitliche Längsränder 16 im entfalteten Zustand über wenigstens 300 mm, insbesondere über wenigstens 400 mm, insbesondere über wenigstens 500 mm, insbesondere über wenigstens 600 mm hinaus. Im dargestellten Fall der Figur 1 ist der einstückige Materialabschnitt 8 beidseits einer Windellängsrichtung 18 um Falzlinien 20 mehrfach auf sich selbst gefaltet. Das jeweils freie Ende 22 des Materialabschnitts 8 bildet einen über die Längsseitenränder 16 um wenigstens 10 mm vorstehenden Anfassbereich 24 zum Ergreifen mit den Fingern eines Benutzers. Dort ist auch ein Verschlusselement 26 an einem freien Ende 22 vorgesehen, und zwar beispielsweise in Form einer klebenden oder vorzugsweise mechanische Verschlusselemente aufweisenden Lasche, welche mit einem Auftreffbereich oder Gegenverschlusselementen am anderen Ende 22 des Hüftgürtels 10 lösbar haftend zusammenwirken kann, wenn der Hüftgürtel 10 zur Bildung einer in Hüftumfangsrichtung geschlossenen Hüftöffnung geschlossen wird.

Die aufeinander gefalteten Teilabschnitte 30 des einstückigen Materialabschnitts 8 sind lösbar aufeinander fixiert, und zwar durch eine Anzahl von im Wesentlichen punktförmigen Fügestellen 32 in Form von vorzugsweise Ultraschallschweißpunkten.

Zum Anlegen der Gürtelwindel 2 wird der Hüftgürtel 10 entfaltet und über das Verschlusselement 26 und den entsprechend ausgebildeten Auftreffbereich am anderen freien Ende des Hüftgürtels 10 auf sich selbst geschlossen. Der Benutzer oder eine Pflegeperson holt nun das im allgemeinen frei nach unten hängende Ende des Hauptteils zwischen den Beinen des Benutzers hervor und befestigt es lösbar an dem ringförmig geschlossenen Hüftgürtel 10, und zwar vorzugsweise auf der vom Benutzer abgewandten Außenseite des Hüftgürtels 10. Der Hauptteil 4 kann wiederum über an sich beliebige klebend oder mechanisch wirkende Verschlussmittel 34, im dargestellten Fall über seitlich vom Hauptteil vorstehende Verschlusslaschen 36 lösbar festgelegt werden. Zusätzlich können in einem entsprechenden Überdeckungsbereich 38, vorzugsweise an der körperzugewandten Innenseite des Hauptteils 4, Verschlussmittel im weitesten Sinn und in an sich beliebiger Ausführung vorgesehen sein.

Die Figuren 2 und 3 zeigen verschiedene Faltungen des einstückigen Materialabschnitts 8 (ohne Darstellung des Verschlusselements 26). Die Faltung nach Figur 2 umfasst zwei Falzlinien 20 auf jeder Seite; eine jeweilige Faltung ist also Z-förmig. Die Gesamtlänge des Hüftgürtels 10 (einschließlich der Quererstreckung über den Hauptteil, also die Umfangslänge der gesamten Hüftöffnung) beträgt ca. 1200 mm (ein etwaiger Überlappungsbereich beim Schließen des Hüftgürtels 10 ist dabei nicht berücksichtigt). Die Gürtelbreite, also deren Erstreckung in Längsrichtung 18, beträgt zwischen 50 und 100 mm.

Die in Figur 3 dargestellte Faltung umfasst auf jeder Seite vier Falzlinien 20. Die gesamte Gürtellänge beträgt ca. 1600 mm.

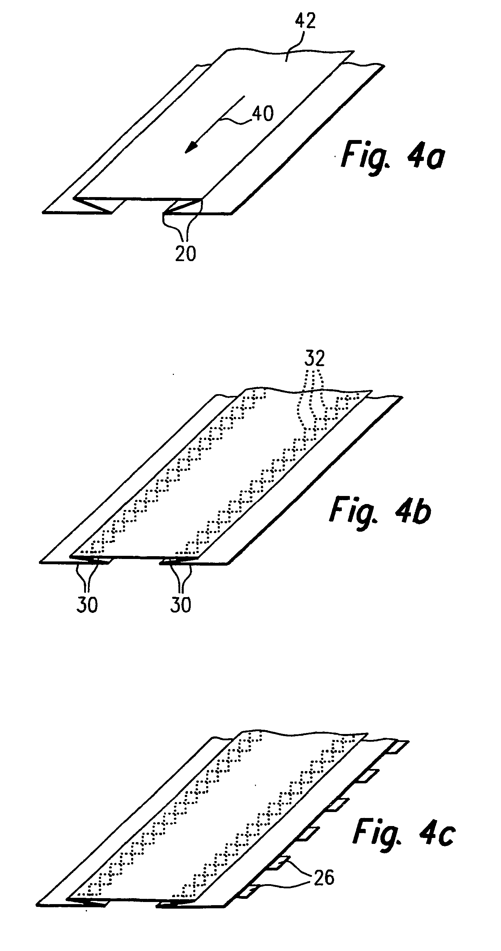

Die Figuren 4a bis 4e verdeutlichen das Herstellungsverfahren für eine erfindungsgemäße Gürtelwindel, und zwar die Herstellung des Hüftgürtels 10. Hierfür wird eine in einer Maschinenlängsrichtung 40 endlose Flachmaterialbahn 42 zugeführt. Die Flachmaterialbahn 42 wird im dargestellten Fall der Figur 4a beispielhaft um zwei Falzlinien 20, also Z-förmig, gefaltet. Auch eine Faltung um drei oder vier oder mehr Falzlinien wäre denkbar. Die aufeinander gefalteten Teilabschnitte 30 werden, wie aus Figur 4b schematisch ersichtlich, lösbar aneinander fixiert. Dies kann in vorteilhafter Weise durch im Wesentlichen punktförmige Fügestellen 32, insbesondere durch Ultraschallschweißpunkte, ausgeführt werden. Figur 4c zeigt die Anfügung von Verschlusselementen 26 an einer Seite der gefalteten Flachmaterialbahn 42.

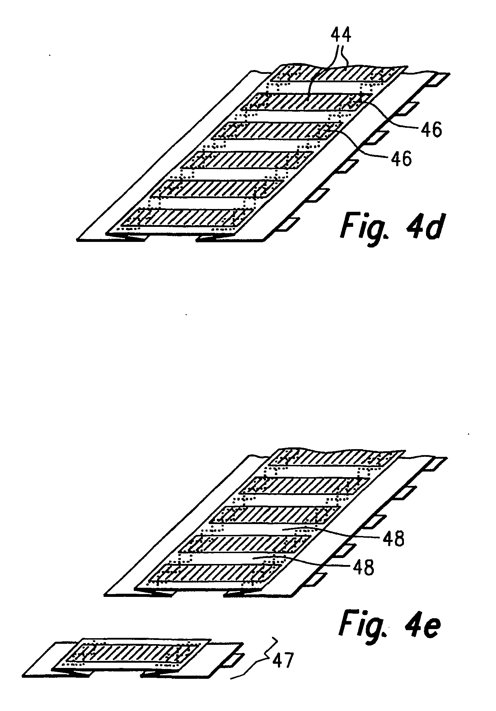

Sodann wird, wie aus Figur 4d ersichtlich ist, ein bereichsweiser Kleberauftrag 44 in in Längsrichtung 40 voneinander beabstandeten Bereichen 46 der Flachmaterialbahn 42 in einem vorzugsweise getakteten Verfahren aufgebracht.

Wie Figur 4e verdeutlicht, werden dann Längsabschnitte 47 quer zur Längsrichtung 40 von der endlos zugeführten Flachmaterialbahn 42 abgetrennt, welche den in Querrichtung einstückig durchgehenden Materialabschnitt 8 zur Bildung des Hüftgürtels 10 darstellen. Man erkennt aus Figur 4e, dass der Schnitt durch kleberfreie Bereiche 48 geführt wird.

Der so abgetrennte Längsabschnitt 47 der Flachmaterialbahn 42 wird dann vorzugsweise auf die Außenseite 12 eines Windelhauptteils 4 unlösbar aufgebracht, so wie dies in Figur 1 dargestellt ist und bereits beschrieben wurde.

## Patentansprüche

1. Wegwerfwindel (2), insbesondere zur Inkontinentenversorgung, mit einem eine in Umfangsrichtung geschlossene Hüftöffnung der Windel bildenden Hüftgürtel (10), der an wenigstens einer Stelle öffenbar und auf sich selbst schließbar ist, und mit einem einen Vorderbereich, einen Rückenbereich und einen dazwischenliegenden Schrittbereich aufweisenden Windelhauptteil (4), der einen Absorptionskörper (6) für Flüssigkeiten aufweist, wobei der Hüftgürtel (10) von einem einstückigen Materialabschnitt (8) gebildet ist, der unlösbar an eine Außenseite (12) des Windelhauptteils (4) angefügt ist und beidseits einer Längsmittelachse um in einer Längsrichtung (18) der Windel erstreckte Falzlinien (20) auf sich selbst gefaltet ist, **dadurch gekennzeichnet, dass** der einstückige Materialabshnitt (8) in gefalteter Konfiguration mit einem Ansfassbereich (24) an seinem freien Ende (22) über einen Lägsseitenrand (16) des windelhauptteils (4) in Querrichtung (14) um insbesondere wenigstens 10 mm vorsteht und dass der Windelhauptteil (4) mit dem Längsende seines Vorderbereichs oder seines Rückenbereichs über erste Verschlussmittel (34) lösbar am Hüftgürtel (10) auf der vom Benutzer abgewandten Außenseite des Hüftgürtels (10) festlegbar ist, derart, dass ein Benutzer bei angelegtem Hüftgürtel (10) den Windelhauptteil (4) zwischen den Beinen hervorholen und das noch freie Längsende des Windelhauptteils (4) am Hüftgürtel (10) außen lösbar festlegen kann.

2. Wegwerfwindel nach Anspruch 1, **dadurch gekennzeichnet, dass** der Hüftgürtel (10) in der gefalteten Konfiguration lösbar fixiert ist.

3. Wegwerfwindel nach Anspruch 2, **dadurch gekennzeichnet, dass** die lösbare Fixierung durch Fügestellen (32) oder Fügebereiche zwischen den aufeinander gefalteten Teilabschnitten (30) des den Hüftgürtel (10) bildenden Materialabschnitts (8) gebildet ist.

4. Wegwerfwindel nach Anspruch 2 oder 3, **dadurch gekennzeichnet, dass** die Fügestellen (32) oder Fügebereiche im Wesentlichen punktförmig sind.

5. Wegwerfwindel nach einem oder mehreren der vorstehenden Ansprüche, **dadurch gekennzeichnet, dass** der einstückige Materialabschnitt (8) beidseits einer Längsmittelachse der Windel um jeweils mindestens zwei Falzlinien (20) auf sich selbst gefaltet ist.

6. Wegwerfwindel nach einem oder mehreren der vorstehenden Ansprüche, **dadurch gekennzeichnet, dass** der einstückige Materialabschnitt (8) beidseits einer Längsmittelachse der Windel um jeweils drei oder vier Falzlinien (20) auf sich selbst gefaltet ist

7. Wegwerfwindel nach einem oder mehreren der vorstehenden Ansprüche, **dadurch gekennzeichnet, dass** der einstückige Materialabschnitt (8) in gefalteter Konfiguration mit einem Anfassbereich (24) an seinem freien Ende (22) über einen Längsseitenrand (16) des Windelhauptteils (4) in Querrichtung (14) um wenigstens 20 mm und weiter insbesondere wenigstens 30 mm vorsteht.

8. Wegwerfwindel nach einem oder mehreren der vorstehenden Ansprüche, **dadurch gekennzeichnet, dass** der einstückige Materialabschnitt (8) in gefalteter Konfiguration mit einem Anfassbereich (24) an seinen beiden freien Enden (22) über einen Längsseitenrand (16) des Windelhauptteils (4) in Querrichtung (14) vorsteht.

9. Wegwerfwindel nach einem oder mehreren der vorstehenden Ansprüche, **dadurch gekennzeichnet, dass** die Erstreckung des den Hüftgürtel (10) bildenden Materialabschnitts (8) im entfalteten Zustand in Querrichtung (14) über den Längsrand (16) des Hauptteils (4) hinaus wenigstens 200 mm, insbesondere wenigstens 300 mm, insbesondere wenigstens 400 mm, insbesondere wenigstens 500 mm und weiter insbesondere wenigstens 600 mm beträgt.

10. Wegwerfwindel nach einem oder mehreren der vorstehenden Ansprüche, **dadurch gekennzeichnet, dass** der Hüftgürtel (10) eine Erstreckung in Längsrichtung (18) von 30 bis 120 mm, insbesondere von 30 bis 100 mm, insbesondere von 30 bis 80 mm, insbesondere von 30 bis 75 mm, insbesondere von 44 bis 70 mm, insbesondere von 40 bis 65 mm und weiter insbesondere von 40 bis 60 mm aufweist.

11. Wegwerfwindel nach einem oder mehreren der vorstehenden Ansprüche, **dadurch gekennzeichnet, dass** der einstückige Materialabschnitt (8) mittels eines insbesondere nur bereichsweisen Kleberauftrags (44) an den Windelhauptteil (4) angefügt ist.

## Claims

1. Disposable diaper (2), in particular for incontinent care, with a hip belt (10) forming a hip opening of the diaper which is closed in peripheral direction which hip belt (10) can be opened and closed onto itself at at least one location, and with a main diaper section (4) having a front region, a rear region and a crotch region disposed between the front region and the rear region, the main diaper section (4) having an absorption body (6) for liquids, wherein the hip belt (10) is formed of a single piece material section (8) which is inseparably attached to an outer side (12) of the main diaper section (4), and which is folded onto itself along folding lines (20) extending in a longitudinal direction (18) on both sides of a longitudinal middle axis, **characterized in that** in the folded configuration, the single piece material section (8) protrudes with a grasping region (24) located on its free end (22) in a transverse direction (14) past a longitudinal side edge (16) of the main diaper section (4), in particular by at least 10 mm, and that the main diaper section (4) can be secured to the hip belt (10) on the outer side of the hip belt (10) facing away from the user in a detachable fashion with the longitudinal end of its front region or of its rear region via closing means (34) in such a fashion that the user, with the hip belt applied (10), can lift the main diaper section (4) from between the legs and can attach the free longitudinal end of the main diaper section (4) to the hip belt (10) outer side in a detachable fashion.

2. Disposable diaper of claim 1, **characterized in that** the hip belt (10) is fixed in the folded configuration in a detachable fashion.

3. Disposable diaper of claim 2, **characterized in that** the detachable fixing is effected by means of attachment locations (32) or attachment regions between the partial sections (30) of the material section (8) forming the hip belt (10) which are folded on top of each other.

4. Disposable diaper of claim 2 or 3, **characterized in that** the attachment locations (32) or attachment regions are substantially point shaped.

5. Disposable diaper according to any one or more of the preceding claims, **characterized in that** the single piece material section (8) on each side of a longitudinal middle axis of the diaper is folded onto itself about at least two respective fold lines (20).

6. Disposable diaper according to any one or more of the preceding claims, **characterized in that** the single piece material section (8) on each side of a longitudinal middle axis of the diaper is folded onto itself about three or respective four fold lines (20).

7. Disposable diaper according to any one or more of the preceding claims, **characterized in that**, in the folded configuration, the single piece material section (8) protrudes with a grasping region (24) located on its free end (22) in a transverse direction (14) past a longitudinal side edge (16) of the main diaper section (4) by at least 20 mm, and further particularly by at least 30 mm.

8. Disposable diaper according to any one or more of the preceding claims, **characterized in that**, in the folded configuration, the single piece material section (8) protrudes with a grasping region (24) at each of its two free ends (22) in a transverse direction (14) past a longitudinal side edge (16) of the main diaper section (4).

9. Disposable diaper according to any one or more of the preceding claims, **characterized in that** the material section (8) forming the hip belt (10) extends in the unfolded state in a transverse direction (14) past the longitudinal edge (16) of the main section (4) by at least 200 mm, in particular by at least 300 mm, in particular by at least 400 mm, in particular by at least 500 mm, and most particularly by 600 mm.

10. Disposable diaper according to any one or more of the preceding claims, **characterized in that** the hip belt (10) has an extension in longitudinal direction (18) of 30 to 120 mm, in particular of 30 to 100 mm, in particular of 30 to 80 mm, in particular of 30 to 75 mm, in particular of 44 to 70 mm, in particular of 40 to 65 mm, and further in particular of 40 to 60 mm.

11. Disposable diaper according to any one or more of the preceding claims, **characterized in that** the single piece material section (8) is attached to the main diaper section (4) with the application of glue (44), preferably in certain regions only.

## Revendications

1. Couche jetable (2), à utiliser en particulier contre l'incontinence, comprenant une ceinture de hanches (10) constituant, pour la couche, une ouverture fermée dans le sens du tour des hanches, ladite ceinture pouvant être ouverte en au moins un point et refermée sur elle-même, ainsi qu'une partie principale de couche (4) présentant une zone avant, une zone arrière et une zone d'entrejambe intermédiaire, ladite partie principale présentant un corps (6) absorbant les liquides, la ceinture de hanches (10) étant constituée par une section de matière monobloc (8), reliée indétachable à une face extérieure (12) de la partie principale (4) de la couche et repliée sur elle-même des deux côtés d'un axe médian longitudinal le long de lignes de pliage (20) s'étendant dans un sens longitudinal (18) de la couche, **caractérisée en ce que** la section de matière monobloc (8) fait saillie en configuration repliée avec une zone de liaison (24) sur son extrémité libre (22) d'un bord latéral longitudinal (16) de la partie principale (4) de la couche dans la direction transversale (14) en particulier d'au moins 10 mm, et **en ce que** la partie principale (4) de la couche peut être fixée, de façon détachable, à la ceinture de hanches (10) par l'extrémité longitudinale de sa zone avant ou de sa zone arrière par l'intermédiaire de premiers moyens de fermeture (34) sur la face extérieure de la ceinture de hanches (10) opposée à l'utilisateur, de sorte que, lorsque la ceinture de hanches (10) est en place, un utilisateur peut faire passer la partie principale (4) de la couche par l'entrejambe et fixer, de façon détachable à l'extérieur, l'extrémité longitudinale encore libre de la partie principale (4) de la couche à la ceinture de hanches (10).

2. Couche jetable selon la revendication 1, **caractérisée en ce que** la ceinture de hanches (10) est fixée détachable en repliée.

3. Couche jetable selon la revendication 2, **caractérisée en ce que** la fixation détachable est formée par des points d'assemblage (32) ou des zones d'assemblage entre les sections partielles (30) repliées les unes sur les autres de la section de matière (8) constituant la ceinture de hanches (10).

4. Couche jetable selon la revendication 2 ou 3, **caractérisée en ce que** les points d'assemblage (32) ou les zones d'assemblage sont sensiblement en forme de points.

5. Couche jetable selon l'une ou plusieurs des revendications précédentes, **caractérisée en ce que** la section de matière monobloc (8) est repliée sur elle-même des deux côtés d'un axe longitudinal médian des couches le long de respectivement au moins deux lignes de pliage (20).

6. Couche jetable selon l'une ou plusieurs des revendications précédentes, **caractérisée en ce que** la section de matière monobloc (8) est repliée sur elle-même des deux côtés d'un axe longitudinal médian de la couche le long de respectivement trois ou quatre lignes de pliage (20).

7. Couche jetable selon l'une ou plusieurs des revendications précédentes, **caractérisée en ce que** la section de matière monobloc (8), lorsqu'elle se trouve en configuration repliée, fait saillie avec une zone de liaison (24) sur son extrémité libre (22) d'un bord latéral longitudinal (16) de la partie principale (4) de la couche dans la direction transversale (14) d'au moins 20 mm et en particulier d'au moins 30 mm.

8. Couche jetable selon l'une ou plusieurs des revendications précédentes, **caractérisée en ce que** la section de matière monobloc (8), lorsqu'elle se trouve en configuration repliée, fait saillie, avec une zone de liaison (24) sur ses deux extrémités libres (22), d'un bord latéral longitudinal (16) de la partie principale (4) de la couche dans la direction transversale (14).

9. Couche jetable selon l'une ou plusieurs des revendications précédentes, **caractérisée en ce que** l'étendue de la section de matière (8) constituant la ceinture de hanches (10) dans l'état déplié dans la direction transversale (14) au-delà du bord longitudinal (16) de la partie principale (4) atteint au moins 200 mm, en particulier au moins 300 mm, en particulier au moins 400 mm, en particulier au moins 500 mm et en particulier au moins 600 mm.

10. Couche jetable selon l'une ou plusieurs des revendications précédentes, **caractérisée en ce que** la ceinture de hanches (10) présente une étendue dans la direction longitudinale (18) de 30 à 120 mm, en particulier de 30 à 100 mm, en particulier de 30 à 80 mm, en particulier de 30 à 75 mm, en particulier de 44 à 70 mm, en particulier de 40 à 65 mm et en particulier de 40 à 60 mm.

11. Couche jetable selon l'une ou plusieurs des revendications précédentes, **caractérisée en ce que** la section de matière monobloc (8) est reliée à la partie principale (4) de la couche au moyen d'une colle (44) appliquée en particulier uniquement par endroits.
